# EUROPEAN PATENT APPLICATION

(11) **EP 4 635 559 A1**
(43) Date of publication of application: **22.10.2025**
(21) Application number: 24177516.2
(22) Date of filing: 23.05.2024
(51) Int. Cl.: A61N 1/39

(54) **A DEFIBRILLATION MONITOR**

(30) Priority: 17.04.2024 WO PCT/CN2024/088167
(71) Applicant: Koninklijke Philips N.V., 5656 AG Eindhoven (NL)
(72) Inventor: WU, Jikun, Eindhoven (NL); ZHANG, Yuyang, Eindhoven (NL); YANG, Chao, 5656AG Eindhoven (NL); CHEN, Chaobin, Eindhoven (NL)
(74) Representative: Philips Intellectual Property & Standards

(57) **Abstract**

A rotatable cover for covering one or more interface elements of a defibrillator monitor. The rotatable cover is rotatable so as to controllably cover and expose the interface element(s).

## Description

### FIELD OF THE INVENTION

The present invention relates to the field of defibrillators, and in particular to defibrillation monitors.

### BACKGROUND OF THE INVENTION

Defibrillators are becoming increasingly commonplace, both in medical environments and the wider public environment. A defibrillator requires a defibrillator monitor for monitoring the subject undergoing defibrillation and/or controlling any defibrillation performed by the defibrillator.

Existing defibrillators comprise at least one interface element for connecting to an external connector. Examples of interface elements include interface elements of a CO₂ module, in which each interface element is adapted to connect to a fluidic connector that carries CO₂ (into or out of the defibrillator device). Other examples of interface elements include interface elements of an ECG module, in which each interface element is an electrical interface element for connecting to one or more electrodes of an ECG system.

To reduce a risk of foreign material ingress into the interface element(s) when not in use, it is common for a defibrillator monitor to comprise a cover that covers the interface element(s). Historically, this cover is configured to slide along an axis to move between a covering position, in which the interface element(s) is/are covered, and an uncovering position, in which the interface element(s) is/are exposed.

There is an ongoing desire to make defibrillator monitors more robust, e.g., less sensitive to damage and/or movement.

### SUMMARY OF THE INVENTION

The invention is defined by the claims.

According to examples in accordance with an aspect of the invention, there is provided a defibrillation monitor comprising: at least one interface element configured to connect to an external connector; and a rotatable cover configured to rotate between: a first orientation, in which the rotatable cover covers the at least one interface element; and a second orientation, in which the rotatable cover exposes the at least one interface element for connection to the external connector.

The present disclosure recognizes that a defibrillation monitor would benefit from covering one or more interface elements (e.g., when not in use), for instance, to protect against the ingress of foreign material into the interface element and/or defibrillation monitor. In particular, it has been recognized that a rotatable cover, that rotatably covers and exposes the interface element(s), will occupy a significantly smaller space than other forms of covers, particularly slidable covers.

Thus, by providing a rotatable cover for the interface element(s), the amount of space occupied by the interface element(s) and (rotatable) cover is significantly reduced compared to defibrillator monitors with existing covers. In approaches in which the interface element and cover are positioned on a housing of the defibrillator monitor, this allows for the housing to have a larger, continuous area, thereby improving the robustness of the defibrillation monitor.

It will be appreciated that the defibrillation monitor may further comprise one or more further interface elements, which are not necessarily covered by a respective (rotatable) cover. Similarly, it will be appreciated that the defibrillation monitor may comprise a plurality of rotatable covers, each configured to rotatably cover and expose a respective set of two or more interface elements. In such examples, each rotatable cover may be embodied as any herein proposed rotatable cover.

In some examples, the at least one interface element comprises at least two interface elements. Thus, a single rotatable cover may be able to selectively cover at least two interface elements. This advantageously reduces a number of covers that need to be provided to cover multiple interface elements, (e.g., rather than having a respective cover for each individual interface element that needs to be covered).

In some examples, the at least one interface element comprises an inlet and an outlet for a fluid carried by a respective fluidic connector. The fluid may be carbon dioxide.

In some examples, the rotatable cover comprises: a central portion configured to rotate about a central axis; and for each interface element, a respective protruding portion that extends, in a respective direction perpendicular to the central axis, from the central portion to, when the rotatable cover is in the first orientation, cover said interface element. This approach provides a dedicated portion of the rotatable cover for each interface element that is separated from one another (by the central section). This reduces a risk that any foreign material ingress will affect multiple interface elements at a same time, as each interface element will be separately covered.

In some examples, the at least one interface element comprises a first interface element and a second interface element; and the respective protruding portion for the first interface element extends along a same axis as the respective protruding portion of the second interface element. This approach helps distance the two protruding portions from one another to further reduce a risk that any foreign material ingress will affect multiple interface elements at a same time, as each interface element will be separately covered.

In some embodiments, rotatable cover comprises a tapered portion between the respective protruding portion for the first interface element and the respective protruding portion for the second interface element. The use of a tapered portion provides a region or area for a user to grip in order to improve an ease of performing manual rotation of the rotatable cover. The use of a tapered portion also reduces an amount of material used to form the rotatable cover, thereby saving material and reducing weight.

In some examples, the at least one interface elements comprises only the first interface element and the second interface element.

In some examples, the defibrillation monitor further comprises a body element comprising an engagement element for engaging with the rotatable cover, wherein the rotatable cover, when engaged with the engagement element, is able to rotate about the engagement element to rotate between the first orientation and the second orientation. The body element provides an intermediate element between the rotatable cover and the remainder of the defibrillation monitor. The body element is thereby able to provide additional structural support and/or protection to the rotatable cover, to reduce a risk of the rotatable cover breaking.

The body element may further comprise, for each interface element, a respective aperture having bounds that surrounds the said interface element. The use of the aperture(s) for the body element provides a guide for an external connector towards the interface element, improving an ease of connecting the external connector to the interface element. Moreover, by surrounding each interface element, the body element is also able to provide additional protection to the rotatable cover (which needs to be able to cover or lie over each interface element).

The body element may further comprise, for at least one interface element, a projection that extends from the bounds of the respective aperture towards the interface element for guiding the respective external connector to the interface element. Each projection increases an ease of connecting any external connector to the interface element by acting as a physical guide for locating the external connector to the interface element.

The defibrillation monitor may comprise a housing element, wherein the body element is configured to engage with the housing element. The housing element may be formed from a portion of a housing of the defibrillation monitor and/or a panel (such as a measurement panel) that connects or couples to a housing of the defibrillation monitor.

The body element may comprise two or more clipping elements for clipping the body element to the housing element. This provides a reliable and intuitive mechanism for securing the body element to the housing element. In preferred examples, the body element comprises at least three clipping elements, e.g., four or more clipping elements.

The defibrillation monitor may further comprise a biasing element configured to bias the rotatable cover towards the first orientation. This approach maintains the rotatable cover in the first orientation (e.g., when not in use), thereby improving a protection of the interface element(s). In particular, by biasing the rotatable cover to the first orientation, a risk that the interface element(s) will become unintentionally exposed to foreign material is significantly reduced.

The biasing element may comprise a torsion spring. This provides a reliable and compact mechanism for biasing the rotatable cover towards the first orientation.

The torsion spring may comprise a first end fixed to a position distanced from the at least one interface element; and a second end configured to rotate with the rotatable element. Preferably,
when the rotatable element is at the first orientation, the second end is over and/or directed/pointing towards the at least one interface element and; when the rotatable element is at the second orientation, the second end is distanced from the at least one interface element. This provides an approach for more reliable and effective biasing of the rotatable cover towards the first orientation.

The defibrillation monitor may further comprise a limiting element configured to restrict the rotation of the rotatable cover: in a first rotational direction from the first orientation to the second orientation, beyond the second orientation; and/or in a second rotational direction from the second orientation to the first orientation, beyond the first orientation.

This advantageously prevents over-rotation of the rotatable cover, thereby reducing a risk of damaging the rotatable cover and/or any further elements of the defibrillation monitor (such as the biasing element, if present).

In some examples, each interface element is configured to receive the electrical connector along a respective interface axis. In such examples, the rotatable element may be correspondingly configured to rotate about a rotation axis parallel to one or each interface axis. This approach significantly reduces a space occupied by the rotatable element, e.g., by avoiding the rotatable element from occupying additional space outside of the volume occupied by the defibrillation monitor.

Put another way, if each interface element within a respective interface plane, then the rotatable cover may be configured to rotate within a cover plane that lies parallel to one or each interface plane, i.e., about an axis perpendicular or normal to one or each interface plane.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an existing defibrillator monitor;
Figs 2 and 3 illustrate a measurement panel for the existing defibrillator monitor;
Figs 4 and 5 illustrates a measurement panel comprising a proposed rotatable cover;
Fig. 6 illustrates a proposed defibrillator monitor;
Fig. 7 provides a perspective view of an upper side of a cover arrangement comprising the proposed rotatable cover;
Fig. 8 provides a perspective view of a lower side of the cover arrangement;
Fig. 9 provides an exploded view of the cover arrangement; and
Fig. 10 provides another view of the lower side of the cover arrangement.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a rotatable cover for covering one or more interface elements of a defibrillator monitor. The rotatable cover is rotatable so as to controllably cover and expose the interface element(s).

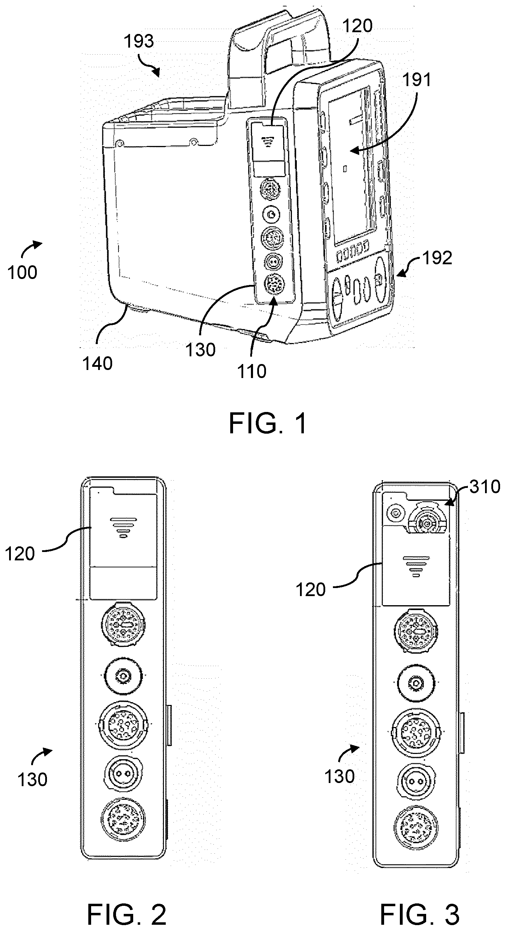

Fig. 1 illustrates an existing defibrillation monitor 100 for improved contextual understanding. The defibrillation monitor 100 may be configured to, when appropriately connected to a subject, control, deliver and monitor defibrillation to a (medical) subject, as well as to monitor one or more physiological parameters of the subject.

The defibrillation monitor 100 comprises a set 110 of interface elements and a cover 120. Each interface element is configured to connect to an external connector.

In the illustrated example, the set 110 of interface elements and the cover 120 are together formed as part of a measurement panel 130 of the defibrillation monitor 100. The measurement panel 130 is positioned within a cutout of the housing 140 of the defibrillation monitor 100.

However, the use of a measurement panel 130 is not essential. In other examples, the set 110 of interface elements and the cover 120 are located directly on the housing 140 of the defibrillator monitor. It is noted that the use of a measurement panel 130 is advantageous, at least for the purposes of maintenance (as it can be removed for access to the defibrillator monitor and/or the interface elements) and/or modularity of the defibrillation monitor.

A non-exhaustive set of examples of suitable interface elements are hereafter described.

Any one or more of the interface elements may comprise a fluidic interface, e.g., an inlet or outlet, for fluidically connecting to a respective fluidic connector. Thus, the set 110 of interface elements may comprise an inlet and an outlet for a fluid carried by a respective fluidic connector.

As another example, any one or more of the interface elements may comprise an electrical interface for connection to an electrical connector, such as a plug and/or wire. Examples of suitable electrical connectors for defibrillation monitors are well known in the art. The electrical connector may, for instance, be a power connector for connection to a power supply and/or power source. Other examples of electrical connectors include those used for subject monitoring, e.g., connecting to a sensor for performing physiological monitoring of a subject.

A wide variety of purposes for interface elements of a defibrillation monitor 100 will be apparent to the skilled person. For instance, the interface element(s) may be adapted to facilitate monitoring of one or more physiological parameters, e.g., connect to CO₂ fluidic connectors for monitoring a CO₂ level of a subject or connect to ECG electrodes to facilitate performance of a (e.g., 12-lead) ECG on the subject. As another example, the interface element(s) may simply comprise a power interface for connection to an (external) power source such as a mains supply and/or auxiliary battery.

Other examples will be readily apparent to the skilled person.

The cover 120 is configured to controllably cover at least one interface element. In particular, the cover may be movable between a covering position (as illustrated in Fig. 1) in which the cover 120 covers the at least one interface element and an uncovering position in which the cover exposes the at least one interface element.

The use of a cover 120 reduces a risk of foreign material ingress into the interface element(s) which are covered when the cover 120 is in the covering position. The cover 120 of the existing defibrillation monitor 100 is configured to slide along an axis to move between the covering position and the uncovering position. In this way, the cover 120 is a slidable cover.

Not all interface elements of the defibrillation monitor need to be covered by a/the cover 120. The selection of which interface elements are covered by a/the cover may be dependent upon the specific use case scenario or embodiment, e.g., dependent upon a need to cover the interface element when not in use. For instance, some interface elements may be more sensitive to the ingress of foreign material than other interface elements.

It will be appreciated that the defibrillation monitor may further comprise other elements or features that are common to defibrillation monitors, but have not been described for the sake of conciseness. These elements/features may include, for instance, a screen or monitor 191 for providing visual feedback to an operator; an input user interface for controlling an operation of the defibrillation monitor 192, one or more defibrillation output interfaces 193 for connecting defibrillation paddles and/or electrodes thereto and so on.

Figs 2 and 3 illustrate the measurement panel 130 for improved understanding of the (slidable) cover 120 of the existing defibrillation monitor. In particular, Fig. 2 illustrates the cover 120 in the covering position and Fig. 3 illustrates the cover in the uncovering position, such that at least one interface element 310 is exposed.

The present disclosure recognizes that a slidable cover 120, such as those used in the existing defibrillator monitor previously described, takes up a significant amount of space. In particular, there needs to be sufficient room for the slidable cover to slide away from the interface elements).

This need for space for the slidable cover can affect the structure and/or stability of the housing.

In particular, if the defibrillator monitor comprises a measurement panel 130, then the size of the measurement panel needs to be larger to accommodate the space for sliding of the slidable cover. As the measurement panel is positioned within a cutout of the housing, this decreases an amount of material from which the housing itself can be formed, providing a less robust defibrillator monitor.

In any event, reducing the space occupied by elements of the cover would advantageously provide additional space for positioning of other elements (e.g., other interface elements) and/or facilitate the provision of a more compact defibrillator monitor.

The present invention thereby recognizes that it would be advantageous to facilitate a reduction in the space occupied or needed by a cover for covering one or more interface elements.

An alternative cover is proposed by the present disclosure, namely a rotatable cover. The rotatable cover rotates between a first orientation and a second orientation. When in the first orientation, the interface element(s) is/are covered. When in the second orientation the interface element(s) is/are exposed.

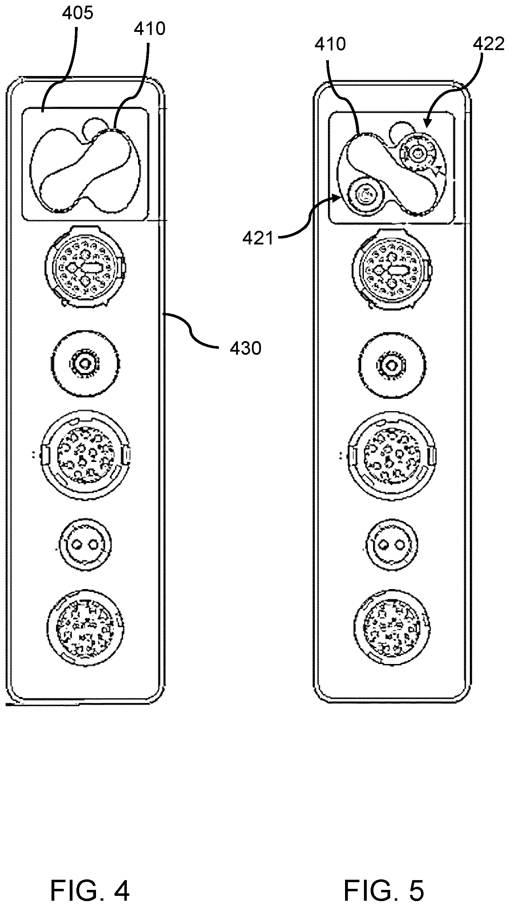

Figs 4 and 5 illustrate an example measurement panel 430 for a defibrillator monitor comprising the proposed rotatable cover 410. The rotatable cover is configured to controllably cover and expose at least one interface element 421, 422. Suitable examples of interface elements have been previously described, and any such examples may be incorporated into hereafter described embodiments.

The rotatable cover 410 may be formed as part of a cover arrangement 405, described in more detail later.

The rotatable cover 410 is configured to rotate between: a first orientation (as illustrated in Fig. 4), in which the rotatable cover covers the at least one interface element; and a second orientation (as illustrated in Fig. 5), in which the rotatable cover exposes the at least one interface element for connection to the/each external connector.

Use of a rotatable cover significantly reduces the amount of space required by the (rotatable) cover to facilitate covering and exposing of the interface element(s), e.g., compared to a slidable cover.

In the illustrated example, the rotatable cover is configured to controllably cover and expose two interface elements. This advantageously performs ingress protection for a plurality of interface elements using a single rotatable cover.

As illustrated in Figs 4 and 5, in preferred examples, each interface element 421, 422 is configured to receive the electrical connector along a respective interface axis. In such examples, the rotatable element 410 is correspondingly configured to rotate about a rotation axis parallel to one or each interface axis.

Put another way, if each interface element 421, 422 lies within a respective interface plane, then the rotatable cover 410 may be configured to rotate within a cover plane that lies parallel to one or each interface plane.

In one example, interface 421 is the CO₂ outlet, while interface 422 is the CO₂ outlet. The interface 421 can be exposed to the air directly, while the interface 422 may be connected with cables or electrical connectors.

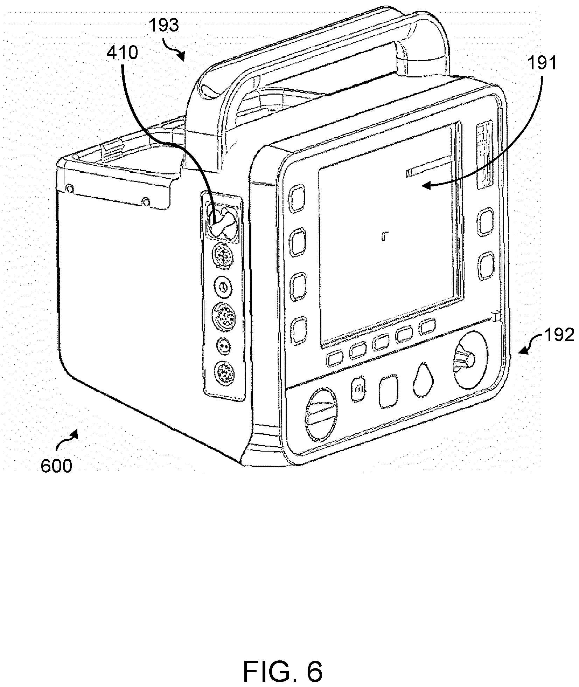

Fig. 6 illustrates a defibrillator monitor 600 comprising the proposed rotatable cover 410. The defibrillator monitor may otherwise be structured and/or comprise further elements of a defibrillator monitor as previously disclosed.

Thus, the defibrillator monitor may, for instance, comprise a screen or monitor 191 for providing visual feedback to an operator; an input user interface for controlling an operation of the defibrillation monitor 192, one or more defibrillation output interfaces 193 for connecting defibrillation paddles and/or electrodes thereto and so on.

Although Figs 4, 5 and 6 illustrate the rotatable cover 410, interface element(s) and cover arrangement (if present) forming part of a measurement panel, in alternative examples, the measurement panel is absent. For instance, the cover arrangement may directly connect or couple to the housing.

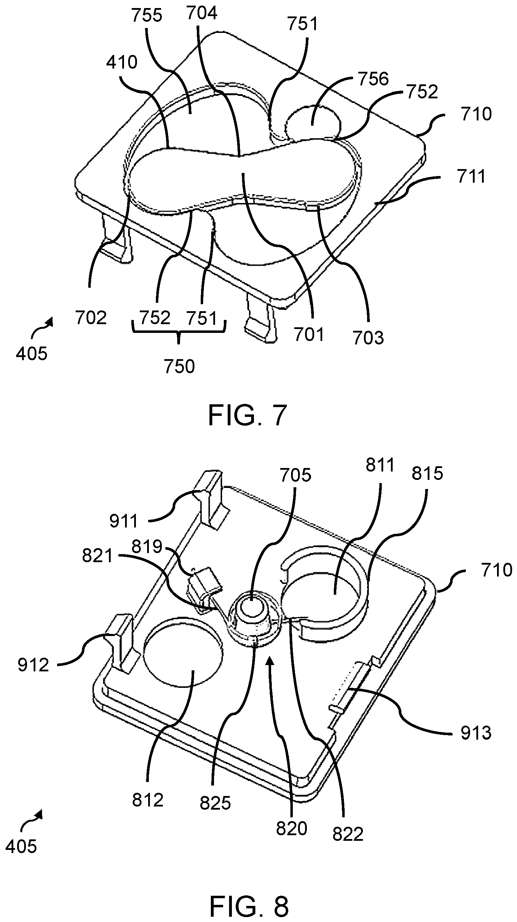

Figs 7, 8, 9 and 10 together illustrate the cover arrangement 405 comprising the rotatable cover 410, together with some additional optional elements. A proposed defibrillator monitor may therefore comprise the cover arrangement 405. The cover arrangement 405 comprises the rotatable cover 410, a body element 710 and a biasing element 720 (the latter two of which are optional).

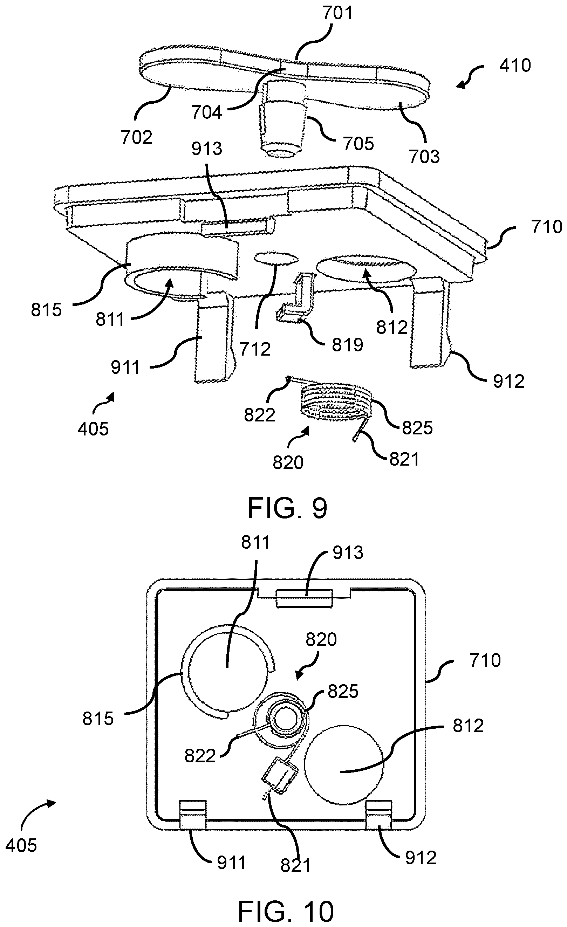

Fig. 7 provides a first perspective view of (an upper surface of) the cover arrangement 405. Fig. 8 provides a second perspective view of (a lower surface of) the cover arrangement 405. Fig. 9 provides an exploded view of the cover arrangement 405. Fig. 10 provides a view of the underside of the cover arrangement 405.

In Figs 7 and 8, the illustrated rotatable cover is in the first orientation. In Fig. 10, the rotatable cover is in the second orientation.

The rotatable cover 420 here comprises a central portion 701 configured to rotate about a central axis (i.e., the cover axis previously mentioned). The rotatable cover comprises a respective protruding portion 702, 703 for each interface element. Each protruding portion is configured to, when the rotatable cover is in the first orientation, cover the corresponding interface element. Of course, when the rotatable cover is in the second orientation, each protruding portion will expose the corresponding interface element.

In the illustrated example, the rotatable cover comprises two protruding portions 702, 703, for covering two interface elements, i.e., a first protruding portion and a second protruding portion.

As illustrated, in some examples, the respective protruding portion for the first interface element extends along a same axis as the respective protruding portion of the second interface element. In this way, the interface elements may be effectively positioned on a diagonal, i.e., diagonally with respect to one another.

In some instances, the rotatable cover comprises a tapered portion 704 between the respective protruding portion 701 for the first interface element and the respective protruding portion 702 for the second interface element. This tapered portion 704 may, for instance, form or contain the central portion 701 of the rotatable cover 420. The tapered portion 704 allows for improved ease of gripping of the rotatable cover by a user or operator, for ease of manually controlling or manipulating the orientation of the rotatable cover.

The body element 710 comprises a body portion 711. In use, the body portion is positioned over the interface element(s) that the rotatable cover 410 is configured to rotatably cover and expose. The body portion may be generally planar, with additional optional elements as later described.

The body element 710 comprises an engagement element 712 configured to engage with the rotatable cover. The rotatable cover, when engaged with the engagement element, is able to rotate about the engagement element to rotate between the first orientation and the second orientation.

In the illustrated example, the rotatable cover 410 comprises a protrusion 705 that extends into an engagement aperture of the body element 710, formed in the body portion 711. The engagement aperture embodies the engagement element. The protrusion 705 engages with the bounds of the engagement aperture 712 and is able to rotate within the engagement aperture. Specifically, when the rotatable cover 410 is engaged with the body element 710, the protrusion 705 will go/pass through the engagement element 712 (e.g., an engagement aperture shown in Fig. 9). As can be seen from Fig. 9, the protrusion 705 comprises a lower protrusion portion and a cylindrical portion and there is a stepped portion between the lower protrusion portion and the cylindrical portion. The cylindrical portion is located between the lower protrusion portion and the tapered portion 704. Further, the lower protrusion portion's circumference is larger than engagement element's 712 circumference, such that the lower protrusion portion will engage with the bounds of the engagement element/aperture 712. The cylindrical portion is sized to flexibly rotate within the engagement element/aperture, such that the engagement between protrusion 705 and the engagement element 714 is secured when those two are engaged with each other. Through the above arrangement, when the protrusion 705 is inserted into the engagement element 712, the lower protrusion portion will penetrate/pass through the engagement element 712 to secure the position of the protrusion 705 to avoid (or at least, mitigate a risk of) detachment between the protrusion 705 and the engagement element 712. Furthermore, the lower protrusion portion will engage with the biasing element 820, as will be described later in this disclosure.

The body element is also able to engage with a housing element of the defibrillator monitor (not visible in any of Figs 7, 8, 9 or 10). The housing element may, for instance, comprise a portion of the housing and/or the measurement panel (if present) of the defibrillator monitor. In this way, the body element may act as an intermediary between the rotatable cover 410 and the housing element.

As perhaps best illustrated in Fig. 9, the body element may comprise two or more clipping elements 911, 912, 913 for clipping the body element to the housing element. The clipping elements are configured to engage with one or more apertures in the housing element (e.g., by way of friction). In particular, each clipping element may be configured to engage with the bounds of one or more apertures in the housing element so as to secure the body element against the housing element. In one example, there are three clipping elements 911, 912 and 913, and the clipping elements 911 and 912, which are configured/positioned at the lower side of the body element, are longer than the element 913, which is configured positioned at the upper side of the body element. When the body element is attached to the housing element, the short clipping element 913 is clipped to the housing element first, and then two longer clipping elements 911 and 912 are clipped to the housing element. This clipping mechanism arrangement facilitates the easy connection between the body element and the house element.

However, the clipping element(s) may be replaced or supplemented with any other mechanism for engaging the body element to a housing element, e.g., one or more screws or the like.

As perhaps best illustrated by Fig. 8, the body element may further comprise for each interface element, a respective aperture 811, 812. Each aperture has bounds that surrounds the said interface element. Each aperture 811, 812 may be formed in the body portion 711 of the body element 710.

Thus, it will be appreciated that the rotatable cover will, when in the first orientation, cover the aperture(s) of the body element and, when in the second orientation, expose the aperture(s) of the body element.

The aperture(s) 811, 812 of the body element may lie in an aperture plane. The rotatable cover is configured to rotate about an axis perpendicular or normal to this aperture plane.

The body element 710 may further comprise, for at least one interface element, a projection 815 that extends from the bounds of the respective aperture towards the interface element. The projection helps to guide the respective external connector to the interface element.

Each projection 815 may surround no less than half of the bounds of the respective aperture. For instance, in the illustrated example, the projection 815 surrounds around 75% of the respective aperture. This approach helps increase a likelihood that the respective external connector will be guided to the interface element and also facilitates the engagement between the projection 815 and the biasing element 820, as described below.

The cover arrangement 405 may further comprise a biasing element 820 configured to bias the rotatable cover 420 towards the first orientation. This approach maintains the rotatable cover in the first orientation (e.g., when not in use), thereby improving the protection of the interface element(s).

In the illustrated example, the biasing element 820 is or comprises a torsion spring. The torsion spring is configured to apply a torque in an opposite direction to a rotation of the rotatable cover from the first orientation to the second orientation. This provides a reliable and consistent approach for biasing the rotatable cover 420 towards the first orientation.

The torsion spring 820 is formed from a first end 821, a coil 825 and a second end 822. The coil connects the first end 821 to the second end 822.

The coil 825 is configured on the rotatable element, e.g., the protrusion 705 of the rotatable element 420, by surrounding the lower protrusion portion of the protrusion 705. The second end is configured to engage with the rotatable element, e.g., the protrusion 705 of the rotatable element 420. For instance, the second end may extend through a passage or tunnel formed in the protrusion 705 of the rotatable element. Through this arrangement, the movement of the second end is limited within the passage or tunnel. Other suitable approaches for connecting the second end 822 of a torsion spring to the rotatable element will be apparent to the appropriately skilled person.

The coil 825 is configured to bias the first end and the second end around the coil 825, according to well-known principles of torsion springs.

In preferred examples, the first end is fixed to a position distanced from the at least one interface element. For instance, the first end may be connected or coupled to a projecting element 819 of the body element 710 that extends outwardly from the (body portion 711 of) the body element 710. The projecting element 819 may, for instance, have the shape of a hook to reduce a risk of the first end decoupling or disconnecting therefrom.

The first and second ends of the torsion spring will move accordingly with the rotation of the rotatable element. In some examples, when the rotatable element is moved from the first orientation to the second orientation, the first end will move along the projecting element 819 but its movement is limited by the projecting element 819, protecting or reducing a risk of any decoupling or disconnecting of the first end. In some examples, when the rotatable element is at the first orientation, the second end is over the interface element 811 (e.g. via the opening of the projection 815). When the rotatable element is at the second orientation, the second end will have moved out of the range of the interface element 811 via the opening of the projection 815 and is distanced from the interface element 811 (e.g., to permit or allow passage of the external connector(s) towards the interface element 811).

This approach helps to correctly bias the rotatable element towards the first orientation.

Other examples of biasing elements will be apparent to the skilled person. For instance, the biasing element may comprise a tension spring configured to pull the rotatable element towards the first orientation. As another example, the biasing element may comprise a piece of elastic material (e.g., a rubber block) configured to push the rotatable element towards the first orientation.

The cover arrangement 405 may further comprise a limiting element 750 configured to restrict the rotation of the rotatable cover.

In particular, the limiting element 750 may comprise a first limiting arrangement 751 configured to restrict (e.g., prevent) rotation of the rotatable cover, in a first rotational direction R1 from the first orientation to the second orientation, beyond the second orientation. In particular, the first limiting arrangement 751 is configured to contact the rotatable cover when the rotatable cover is in the second orientation to thereby physically restrict or prevent a further rotation of the rotatable cover beyond the second orientation (when being rotated from the first orientation to the second orientation). Thus, rotation of the rotatable cover beyond the second orientation may be effectively restricted or prevented.

The first limiting arrangement 751 may, for instance, comprise multiple limiting portions, each configured to contact a different part of the rotatable cover to prevent rotation beyond the second orientation.

Similarly, the limiting element 750 may comprise a second limiting arrangement 752 configured to restrict (e.g., prevent) rotation of the rotatable cover, in a second rotational direction from the second orientation to the first orientation, beyond the first orientation. In particular, the second limiting arrangement 752 is configured to contact the rotatable cover when the rotatable cover is in the first orientation to thereby physically restrict or prevent a further rotation of the rotatable cover beyond the first orientation (when being rotated from the second orientation to the first orientation). Thus, rotation of the rotatable cover beyond the first orientation may be effectively restricted or prevented.

The second limiting arrangement 752 may, for instance, comprise multiple limiting portions, each configured to contact a different part of the rotatable cover to prevent rotation beyond the first orientation.

In the illustrated example, the limiting element 750 of the cover arrangement 405 comprises both the first and the second limiting arrangement. The limiting element is formed from the bounds of an indentation or cavity 755 in the body element 710 (e.g., the body portion 711) against which the rotatable cover engages (when appropriately rotated).

In particular, the rotatable cover 755 is configured to sit within the indentation or cavity 755, i.e., be inset within the indentation or rotatable cavity, e.g., to not extend outside of the indentation or cavity in a direction away from the body element 710. This significantly reduces a risk of the rotatable cover 755 breaking or catching on external objects, as the rotatable cover will be protected by the body element.

In such examples, the body element may further comprise, adjacent to the indentation or cavity 755, a further indentation 756 (e.g. less deep than the first indentation). This further indentation may be formed in the limiting element 750 of the cover element, particularly the second limiting arrangement 752 (if present).

The further indentation is preferably located to be adjacent to the rotatable cover 410 when the rotatable cover is in the first orientation, e.g., such that the further indentation exposes a side surface of the rotatable cover when the rotatable cover is in the first orientation. The further indentation 756 facilitates an increased ease for a user to manually engage with the rotatable cover, e.g., to rotate the rotatable cover. In particular, the further indentation provides a location for a finger or digit of a user to sit against the (side surface of the) rotatable cover 420 for performing a rotation of the rotatable cover.

Another example of a limiting element 750 may be formed from, when present, one or more projections 815 that extend from the body element and a torsion spring 820 (e.g., having a second end coupled to the rotatable cover). In particular, the torsion spring may be configured to engage with the projection(s) when the rotatable cover has rotated to the first orientation and/or second orientation, and prevent or restrict further rotation of the rotatable cover.

Other suitable examples of a limiting element will be readily apparent to the skilled person.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A defibrillation monitor comprising:
at least one interface element configured to connect to an external connector; and
a rotatable cover configured to rotate between:
a first orientation, in which the rotatable cover covers the at least one interface element; and
a second orientation, in which the rotatable cover exposes the at least one interface element for connection to the external connector.

2. The defibrillation monitor of claim 1, wherein the at least one interface element comprises at least two interface elements.

3. The defibrillation monitor of claim 2, wherein the at least one interface element comprises an inlet and an outlet for a fluid carried by a respective fluidic connector.

4. The defibrillation monitor of any one of claims 1 to 3, wherein the rotatable cover comprises:
a central portion configured to rotate about a central axis; and
for each interface element, a respective protruding portion that extends, in a respective direction perpendicular to the central axis, from the central portion to, when the rotatable cover is in the first orientation, cover said interface element.

5. The defibrillation monitor of claim 4, wherein:
the at least one interface element comprises a first interface element and a second interface element; and
the respective protruding portion for the first interface element extends along a same axis as the respective protruding portion of the second interface element.

6. The defibrillation monitor of claim 5, wherein the rotatable cover comprises a tapered portion between the respective protruding portion for the first interface element and the respective protruding portion for the second interface element.

7. The defibrillation monitor of any one of claims 1 to 6, further comprising a body element comprising an engagement element for engaging with the rotatable cover, wherein the rotatable cover, when engaged with the engagement element, is able to rotate about the engagement element to rotate between the first orientation and the second orientation.

8. The defibrillation monitor of claim 7, wherein the body element further comprises, for each interface element, a respective aperture having bounds that surrounds the said interface element.

9. The defibrillation monitor of claim 8, wherein the body element further comprises, for at least one interface element, a projection that extends from the bounds of the respective aperture towards the interface element for guiding the respective external connector to the interface element.

10. The defibrillation monitor of any one of claims 8 or 9, comprising a housing element, wherein the body element is configured to engage with the housing element.

11. The defibrillation monitor of claim 10, wherein the body element comprises two or more clipping elements for clipping the body element to the housing element.

12. The defibrillation monitor of any one of claims 1 to 11, further comprising a biasing element configured to bias the rotatable cover towards the first orientation.

13. The defibrillation monitor of claim 12, wherein the biasing element comprises a torsion spring.

14. The defibrillation monitor of claim 13, wherein the torsion spring comprises:
a first end fixed to a position distanced from the at least one interface element; and
a second end configured to rotate with the rotatable element, wherein:
when the rotatable element is at the first orientation, the second end is over the at least one interface element; and
when the rotatable element is at the second orientation, the second end is distanced from the at least one interface element.

15. The defibrillation monitor of any one of claims 1 to 14, further comprising a limiting element configured to restrict the rotation of the rotatable cover:
in a first rotational direction from the first orientation to the second orientation, beyond the second orientation; and/or
in a second rotational direction from the second orientation to the first orientation, beyond the first orientation.
